Europäisches Patentamt

European Patent Office

Office européen des brevets

(11) Publication number: **0 347 843**
A1

# EUROPEAN PATENT APPLICATION

(21) Application number: **89111217.9**

(51) Int. Cl.⁴: **A61K 31/355 , A61K 47/00**

(22) Date of filing: **20.06.89**

(30) Priority: **24.06.88 JP 156170/88**

(43) Date of publication of application:
**27.12.89 Bulletin 89/52**

(84) Designated Contracting States:
**AT BE CH DE FR GB IT LI NL SE**

(71) Applicant: **Eisai Co., Ltd.**
**6-10, Koishikawa 4-chome Bunkyo-ku**
**Tokyo(JP)**

(72) Inventor: **Tsushima, Yuki**
**10-27, Kashiwa 1-chome**
**Honjou-shi Saitama(JP)**
Inventor: **Tateishi, Kimio**
**4-2, Sakae 2-chome**
**Honjou-shi Saitama(JP)**
Inventor: **Ohsawa, Shigemitsu**
**2286-12, Suehirocho**
**Honjou-shi Saitama(JP)**

(74) Representative: **Hansen, Bernd, Dr.rer.nat. et**
**al**
**Hoffmann, Eitle & Partner Patentanwälte**
**Arabellastrasse 4 Postfach 81 04 20**
**D-8000 München 81(DE)**

(54) **Vitamine E composition improved in absorption.**

(57) A vitamine E composition is improved in absorption and comprises vitamine E or its derivative, lecithin and an unsaturated fatty acid.

EP 0 347 843 A1

## Vitamine E composition improved in absorption

The invention relates to a vitamine E composition being improved in its absorption by the digestic system. It is moreover useful to assure treatment of diseases taking place in the circulating system by improving the absortion of vitamine E contained in a medicine or food.

( Prior arts )

In the state of arts, vitamine E, such as alpha-tocophenol, alpha-tocopheryl acetate and alpha-tocopheryl succinate, is available in the medicine in the form of soft capsules with a binder of a vegitable oil. The vitamine E is soluble in oil and therefore the absorption of it depends on the action of the lever and pancreas. When the bile is not secreting, in particular, the vitamine E is not absorbed so much. See Gallo-Torres, H.E., Lipids, 5, 379(1970). It can be said that the absorption of vitamine E by the gut depends on persons having different situations about secretion of digestive fluids. In addition it is expected that the absorption by a person would be influenced by having eaten food or not just in advance. For example, tocopherol nicotinate, one of the oil-soluble medicine is less absorbed on administering it to a person being hungry than another person just having eaten. See Takeshi FUJITA et al., Yakuri to Chiryo 8(2), 44(1980), a Japanese literature. This is the reason the absorption of vitamine E and its derivative depends on the situation of the digestive activity and food eaten and in addition an invention on manufacturing a vitamine E preparation is needed to improve its absorption.

( Summary of the Invention )

The invention relates to an improvement of the absorption of vitamine E and its derivative administered orally to a living body. This purpose is attained by orally adminstering vitamine E or its derivative together with lecithin and an unsaturated fatty acid.

The invention provides a vitamine E composition, improved in its absorption, which comprises vitamine E or its derivative, lecithin and an unsaturated fatty acid.

It is preferable that a weight ratio of the lecithin to the unsaturated fatty acid ranges from 1:1 to 1:19 and the composition comprises 50 to 400 percent by weight, based on the vitamine E and its derivative, of the lecithin and the unsaturated fatty acid in total.

It is preferable that the unsaturated fatty acid is oleic acid or linoleic acid.

The invention further provides a soft capsule containing the composition as defined above.

( Detailed statement of the invention )

The vitamine E and its derivative to use in the invention include d1-alpha-tocopherol, d-alpha-tocopherol, acetate thereof, succinate thereof, which are available for the therapeutical use, and other tocopherols to be used for food additives.

It is preferred that the lecithin is phosphatidyl choline or a mixture of it and another material, such as yolk lecithin and soy bean lecithin. Purified soy bean lecithin is more preferable to the effect.

The unsaturated fatty acid preferably includes linoleic acid and oleic acid. Oleic acid is more preferable since it is highly soluble in d-alpha-tocopherol and is commertially available in the tradename of Extra Olein 90 and 99 from Nihon Yushi K.K., a Japanese corporation.

It is preferable that a weight ratio of lecithin to an unsaturated fatty acid ranges between 1:1 and 1:19 and the total amount of both ranges from to to 400 percent by weight based on vitamine E and its derivative.

The composition may be obtained in the form of an oily liquid by melting vitamine E or its derivative and dissolving it in a liquid mixture of lecithin and an unsaturated fatty acid. The oily liquid may be further encapsulated to produce soft capsules. Alternatively it may be adsorbed onto hard capsules or porous powder to produce granules or tablets.

The composition may be produced according to conventional processes used for medicine forms. For example, the soft capsules are obtained by mixing a mixture of lecithin and an unsaturated fatty acid with vitamine E or its derivative at 40 to 60 degree C, while agitated, allowing the resultant to get cool and charging soft capsules with the obtained oily solution at 25 degree C according to a conventional method for encapsulation.

The granules and tablets may be obtained by making a porous inorganic powder the above obtained oily solution and molding the resultant into granules or tablets.

The oily solution may further comprises a free fatty acid such as palmitic acid, a vegitable oil and a surfactant such as Tween 80. These additives, not essential to the invention, serve to control the form of vitamine E or its derivative and prevent the crystals from eluding out. These additives are not proposed here by taking into account what influence to put on the absorption by the digestive system. Another additive may be used without lowering the advantage of the invention.

The invention can reduce changes in absorption of vitamine E and its derivative by the digestive system, that is, it can improve the maximum concentration in blood (Cmax), appearing in data on concentration in blood and then an area below a curve showing a concentration in blood (AUC).

( Brief Description of Drawing )

Fig. 1 shows results of Test I.
Fig. 2 shows results of Test II.

( Example of the Composition )

Example 1

150 g of lecithin and 1150 g of oleic acid were added to 1000 g of d-alpha-tocopherol and the mixture was heated up to 50 degree C in nitrogen gas to get to a uniform mixture. It was allowed to cool down to 25 degree C. Soft capsules were charged with the mixture according to a coventional method for encapsulation with a filler for authomatic soft capsules of the Liner type to obtain soft capsules having the content of 230 mg per one capsule. The film-forming component for the capsules comprised 100 g of gelatin, 400 g of concentrated glycerin, 30 g of ethyl paraben and 10 g of propyl paraben.

Example 2

150 g of lecithin and 1150 g of oleic acid were added to 1000 g of dl-alpha-tocopheryl acetate and the mixture was heated up to 50 degree C to obtain a uniform mixture. It was allowed to cool down to 40 degree C. Hard capsules were charged with the mixture with a filler of oily, viscous liquid, having a tradename OP-8, available from Nippon Elanco K.K. to obtain hard capsules having the content of 250 mg per gram.

Test I

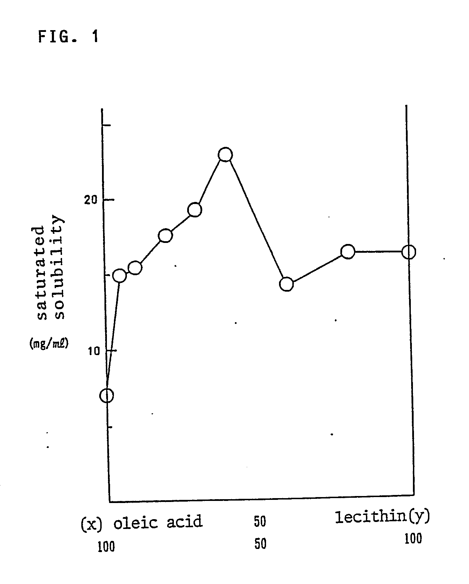

It was here determined how much influence a mixture of oleic acid and lecithin would put on the solubility of d-alpha-tocopherol. It was determined with changing ratios of the oleic acid to the lecithine how to dissolve d-alpha-tocopherol in 20 mM solution of bile acid. Results are shown in Fig. 1 in view of saturated solubility. A high solubility of d-alpha-tocopherol was available when lecithin and oleic acid were present there at their weight ratio of 1:1 to 1:19, compared with the solubility of 2.0 mg/ml when both additives were absent.

Test II

Test samples E-1, E-2 and E-3 were prepared according to the respective methods shown in Table 1. Feed stuff to which vitamine E had been added was fed to beagle dogs. In 30 minutes thereafter, 3 capsules of each test sample with 30 ml of water was orally administered to the beagle dogs. Blood of the beagle dogs were sampled from time to time and a concentration in the blood of d-alpha-tocopherol was determined in the following way, using a cross-over method of three dogs.

The determination started with adding to 0.2 ml of blood serum 1 ml of water and 1 ml of a solution in ethanol of 2 micrograms per millileter of dl-tocol for the internal reference substance and shaking the obtained mixutre for 30 minutes. 5 ml of n-hexane was then added to the mixture and the mixture was further shaked for 20 minutes. It was treated with a centrifugal separation at 3000 rotation per minute for 10 minutes. The phase of the n-hexane was diluted out in nitrogen gas. 0.2 ml of ethanol was added to the residue to form a solution. 20 microliter of the solution was treated with the high performance liquid chromatography with a filler of Nucleosil C18, a carrier liquid of methanol, detected with a fluorescent detector having an exciting wave length of 294 nm and a determining wave length of 340 nm.

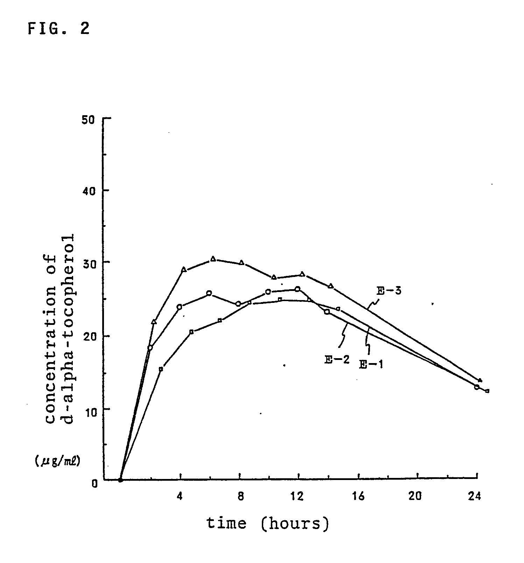

Results of the determination are shown in Fig. 2.in view of increases of concentration in blood from the initial value. The initial value indicates the data obtained just before the administration. The time Tmax when the maximum concentration was available, the maximum concentration Cmax (microgram per milliliter) and the area AUC (microgram.hour per milliliter) per below a concentration curve for a period of 24 hours after the administration are shown in Table 2, being available from the data of Fig. 2.

E-3, falling within the invention, is improved in respect to AUC and Cmax, compared to E-1 of no additive and E-2 of oleic acid only added. It shows that the invention provides an improved absorption of vitamine E.

Table 1

|  |  | comparison | | invention |
|---|---|---|---|---|
| ingredients(mg) |  | E-1 | E-2 | E-3 |
| in the core |  |  |  |  |
| d-alpha-tocopherol |  | 100 | 100 | 100 |
| oleic acid |  | - | 130 | 115 |
| lecithin |  | - | - | 15 |
| in the shell |  |  |  |  |
| gelatin |  | 83.5 | 100 | 100 |
| conc. glycerin |  | 16.7 | 40 | 40 |
| ethyl paraben |  | 0.25 | 0.3 | 0.3 |
| propyl paraben |  | 0.08 | 0.1 | 0.1 |
| water |  | suitable amount | | |
| d-sorbit |  | 8.35 | - | - |
| weight of the shell |  | 110 | 145 | 145 |
| total weight |  | 210 | 375 | 375 |

Table 2

|  | $T_{max}$ (hr) | $C_{max}$ ($\mu g/m\ell$) | AUC ($\mu g \cdot hr/m\ell$) |
|---|---|---|---|
| E-1 | 9.0 ±1.3 | 27.0±6.8 | 469.0 ±119.9 |
| E-2 | 8.0 ±1.8 | 29.5±2.9 | 495.9 ± 53.3 |
| E-3 | 7.0 ±1.7 | 31.3±3.9 | 562.5 ± 81.5 |

Claims

1. A vitamine E composition, improved in absorption, which comprises vitamine E or its derivative, lecithin and an unsaturated fatty acid.

2. A composition as claimed in Claim 1, in which a weight ratio of the lecithin to the unsaturated fatty acid ranges from 1:1 to 1:19.

3. A composition as claimed in Claim 1 or 2, which comprises 50 to 400 percent by weight, based on the vitamine E and its derivative, of the lecithin and the unsaturated fatty acid in total.

4. A composition as claimed in Claim 1, in which the unsaturated fatty acid is oleic acid or linoleic acid.

5. A soft capsule containing the composition as defined in Claim 1.

FIG. 1

FIG. 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, vol. 99, no. 23, 5th December 1983, page 634, abstract no. 193488r, Columbus, Ohio, US; & JP-A-58 146 256 (Q.P. CORP.) 31-08-1983 * Abstract * | 1,4-5 | A 61 K 31/355 A 61 K 47/00 |
| X | CHEMICAL ABSTRACTS, vol. 104, no. 3, 20th January 1986, page 421, abstract no. 18890r, Columbus, Ohio, US; & JP-A-60 153 774 (HOHNEN OIL CO., LTD) 13-08-1985 * Abstract * | 1,4-5 | |

TECHNICAL FIELDS SEARCHED (Int. Cl.4)

A 61 K

The present search report has been drawn up for all claims

| Place of search | Date of completion of the search | Examiner |
|---|---|---|
| THE HAGUE | 26-09-1989 | SCARPONI U. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)